(19)

Europäisches Patentamt
European Patent Office
Office européen des brevets

(11) **EP 3 251 588 A1**

(12) **EUROPEAN PATENT APPLICATION**

(43) Date of publication:
**06.12.2017 Bulletin 2017/49**

(51) Int Cl.:
**A61B 5/00** *(2006.01)*  **A61B 5/024** *(2006.01)*

(21) Application number: **17163197.1**

(22) Date of filing: **28.03.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **31.05.2016 IN 201621018713**

(71) Applicant: **Tata Consultancy Services Limited Mumbai 400 021 Maharashtra (IN)**

(72) Inventors:
• **Bandyopadhyay, Soma**
  **Kolkata - 700160, West Bengal (IN)**

• **Ukil, Arijit**
  **700160 Kolkata, West Bengal (IN)**
• **Puri, Chetanya**
  **Kolkata - 700160 , West Bengal (IN)**
• **Singh, Rituraj**
  **700160 Kolkata, West Bengal (IN)**
• **Pal, Arpan**
  **700160 Kolkata West Bengal (IN)**
• **Murthy, C A**
  **700108 Kolkata, West Bengal (IN)**
• **Mandana, Kayapanda**
  **700107 Kolkata, West Bengal (IN)**

(74) Representative: **Goddar, Heinz J.**
  **Boehmert & Boehmert**
  **Anwaltspartnerschaft mbB**
  **Pettenkoferstrasse 22**
  **80336 München (DE)**

(54) **METHOD AND SYSTEM FOR PHYSIOLOGICAL PARAMETER DERIVATION FROM PULSATING SIGNALS WITH REDUCED ERROR**

(57)     A method and system for deriving physiological parameters from pulsating signals with reduced error is provided. The method is performed by extracting pulsating signals, removing spurious perturbations in the extracted pulsating signals for smoothening of said extracted pulsating signals, deriving local minima points in the smoothened pulsating signal, deriving systolic maxima point between two derived local minimas, deriving most probable pulse duration and most probable peak-to-peak distance using the derived local minima points and the derived systolic maxima points, deriving dicrotic minima while ensuring that every dicrotic minima is preceded by a systolic maxima point and followed by a beat start point of said systolic maxima, deriving diastolic peak while ensuring that every dicrotic maxima is preceded by a diastolic notch and followed by next beat start point of that maxima and deriving physiological parameters from the derived local minima points, systolic maxima points, dicrotic notch and diastolic peak.

```
┌─────────────────────────────────────────┐
│ Signal extraction module                │ 202
└─────────────────────────────────────────┘
                    │
┌─────────────────────────────────────────┐
│ Smoothening module                      │ 204
└─────────────────────────────────────────┘
                    │
┌─────────────────────────────────────────┐
│ Minima derivation module                │ 206
└─────────────────────────────────────────┘
                    │
┌─────────────────────────────────────────┐
│ Maxima derivation module                │ 208
└─────────────────────────────────────────┘
                    │
┌─────────────────────────────────────────┐
│ Statistical learning module             │ 210
└─────────────────────────────────────────┘
                    │
┌─────────────────────────────────────────┐
│ Dicrotic minima derivation module       │ 212
└─────────────────────────────────────────┘
                    │
┌─────────────────────────────────────────┐
│ Diastolic peak derivation module        │ 214
└─────────────────────────────────────────┘
                    │
┌─────────────────────────────────────────┐
│ Physiological parameter derivation module│ 216
└─────────────────────────────────────────┘
```

**Figure 2**

**Description**

**CROSS-REFERENCE TO RELATED APPLICATIONS AND PRIORITY**

The patent application claims to India Application No. 20162108713, filed on 31/05/2016.

**FIELD OF THE INVENTION**

The present application generally relates to biomedical signal processing. More particularly, the application provides a method and system for deriving physiological parameters from pulsating signals with reduced error.

**BACKGROUND**

Huge number of IoT devices are available to promote health care management and wellness. It is undoubted that IoT Healthcare solutions can provide remote monitoring to support patients suffering from various diseases and disorders. But, a gamut of expensive sensor devices, sophisticated, periodic setup, maintenance and calibration as well as up-to-date training are required for such purpose to come to fruition. In order to promote widespread usage and affordability, such costly and extensive intricacies do not work positively towards the ubiquity and success of mobile and preventable health care, specifically in developing countries. As a result, deriving various physiological parameters of a person in a noninvasive and affordable manner is a significant task and challenge.
Pulsating signals have gained high importance for the purpose of derivation of various physiological parameters of a person. As a result, extracting the various time series features of pulsating signals like photoplethysmogram (PPG) signal and then co-relating them with physiological parameters is a necessity to create noninvasive and affordable healthcare analytics applications. However, in the majority of cases, a pulsating signal like photoplethysmogram (PPG) signal has lot of noise and the analytics to be performed are mostly run on low power battery operated devices like mobile phones. Therefore, a feature derivation mechanism with reduced errors and reduced resource usage is an important requirement for effectively deriving various physiological parameters of a person in a noninvasive and affordable manner. Thereby, deriving physiological parameters from pulsating signals with reduced error while requiring low computational power is still considered to be one of the biggest challenges of the technical domain.

**OBJECTIVES OF THE INVENTION**

In accordance with the present invention, an objective is to provide for a method and system for deriving physiological parameters from pulsating signals with reduced error.
Another objective of the invention is to derive physiological parameters from pulsating signals while requiring low computational power.
Yet another objective of the invention is to derive physiological parameters from pulsating signals while maintaining an extremely low level of false negatives.

**SUMMARY OF THE INVENTION**

Before the present methods, systems, and hardware enablement are described, it is to be understood that this invention is not limited to the particular systems, and methodologies described, as there can be multiple possible embodiments of the present invention which are not expressly illustrated in the present disclosure. It is also to be understood that the terminology used in the description is for the purpose of describing the particular versions or embodiments only, and is not intended to limit the scope of the present invention which will be limited only by the appended claims.
The present disclosure envisages a method and system which can derive physiological parameters from pulsating signals with reduced error.
In an embodiment of the invention, a method for deriving a plurality of physiological parameters from pulsating signals with reduced error for monitoring cardiac health of a patient is provided. The method comprises extracting pulsating signals from the patient, removing spurious perturbations in the extracted pulsating signals using two different time window lengths for smoothening of said extracted pulsating signals, deriving local minima points in the smoothened pulsating signals based on the discrete nature of said pulsating signals and strictly rising and falling edge of said pulsating signals and union of minima points derived from the smoothened pulsating signals with two different sizes, deriving systolic maxima point between two derived local minimas based on the discrete nature of said pulsating signals, strictly rising and falling edge of said pulsating signals and union of maxima points derived from the smoothened pulsating signals with two different sizes, deriving most probable pulse duration and most probable peak-to-peak distance using the derived local minima points and the derived systolic maxima points and based on the univariate nature of said pulsating signals, deriving dicrotic minima based on the discrete nature of said pulsating signals, strictly rising and falling edge of said pulsating signals and union of the local minima points derived from two different smoothened pulsating signals with two different sizes ensuring that every dicrotic minima is preceded by a systolic maxima point and followed by a beat start point of said systolic maxima, deriving diastolic peak based on the discrete nature of said pulsating signals, strictly rising and falling edge of said pulsating signals and union of the systolic maxima points derived from two different smoothened pulsating signals with two different sizes ensuring that every dicrotic maxima is preceded by a diastolic notch and followed by next beat start point of that maxima and deriving physiological parameters from the derived local minima points, systolic maxima points, dicrotic notch and diastolic peak.

In another embodiment of the invention, a system (200) for deriving a plurality of physiological parameters from pulsating signals for monitoring cardiac health of a patient is provided. The system (200) comprises of a signal extraction module (202), a smoothening module (204), a minima derivation module (206), a maxima derivation module (208), a statistical learning module (210), a dicrotic minima derivation module (212), a diastolic peak derivation module (214) and a physiological parameter derivation module (216).

## BRIEF DESCRIPTION OF THE DRAWINGS

The foregoing summary, as well as the following detailed description of preferred embodiments, are better understood when read in conjunction with the appended drawings. For the purpose of illustrating the invention, there is shown in the drawings exemplary constructions of the invention; however, the invention is not limited to the specific methods and system disclosed. In the drawings:

Fig 1 shows a flow chart illustrating method for physiological parameter derivation from pulsating signal with reduced error.

Fig. 2 shows a block diagram of a system for physiological parameter derivation from pulsating signal with reduced error.

Fig. 3 shows an exemplary embodiment of physiological parameter derivation from pulsating signal with reduced error.

## DETAILED DESCRIPTION OF THE INVENTION

Some embodiments of this invention, illustrating all its features, will now be discussed in detail.

The words "comprising," "having," "containing," and "including," and other forms thereof, are intended to be equivalent in meaning and be open ended in that an item or items following any one of these words is not meant to be an exhaustive listing of such item or items, or meant to be limited to only the listed item or items.

It must also be noted that as used herein and in the appended claims, the singular forms "a," "an," and "the" include plural references unless the context clearly dictates otherwise. Although any systems and methods similar or equivalent to those described herein can be used in the practice or testing of embodiments of the present invention, the preferred, systems and methods are now described. In the following description for the purpose of explanation and understanding reference has been made to numerous embodiments for which the intent is not to limit the scope of the invention.

One or more components of the invention are described as module for the understanding of the specification. For example, a module may include self-contained component in a hardware circuit comprising of logical gate, semiconductor device, integrated circuits or any other discrete component. The module may also be a part of any software programme executed by any hardware entity for example processor. The implementation of module as a software programme may include a set of logical instructions to be executed by a processor or any other hardware entity.

The disclosed embodiments are merely exemplary of the invention, which may be embodied in various forms.

The elements illustrated in the Figures interoperate as explained in more detail below. Before setting forth the detailed explanation, however, it is noted that all of the discussion below, regardless of the particular implementation being described, is exemplary in nature, rather than limiting. For example, although selected aspects, features, or components of the implementations are depicted as being stored in memories, all or part of the systems and methods consistent with the natural disaster prediction system and method may be stored on, distributed across, or read from other machine-readable media.

Method steps of the invention may be performed by one or more computer processors executing a program tangibly embodied on a computer-readable medium to perform functions of the invention by operating on input and generating output. Suitable processors include, by way of example, both general and special purpose microprocessors. Generally, the processor receives (reads) instructions and data from a memory (such as a read-only memory and/or a random access memory) and writes (stores) instructions and data to the memory. Storage devices suitable for tangibly embodying computer program instructions and data include, for example, all forms of non-volatile memory, such as semiconductor memory devices, including EPROM, EEPROM, and flash memory devices; magnetic disks such as internal hard disks and removable disks; magneto-optical disks; and CD-ROMs. Any of the foregoing may be supplemented by, or incorporated in, specially-designed ASICs (application-specific integrated circuits) or FPGAs (Field-Programmable Gate Arrays). A computer can generally also receive (read) programs and data from, and write (store) programs and data to, a non-transitory computer-readable storage medium such as an internal disk (not shown) or a removable disk.

The present disclosure provides a method and system for deriving physiological parameters from pulsating signals with reduced error.

In an aspect of the invention, photoplethysmogram (PPG) signals can be used as the pulsating signal. The different features derived from PPG signal can be used to detect various parameters to detect physiological issues in a patient.

In an exemplary aspect, once the pulse peak amplitude (PPA) and the systolic amplitude is derived from the PPG signal, premature ventricular contraction in a patient's body can be detected. The height of AC component of the photoplethysmogram is proportional to the pulse pressure, the difference between the systolic and diastolic pressure in the arteries. The PPG pulse for the cardiac cycle with the PVC results in lower amplitude blood pressure and a PPG. Ventricular tachycardia and ventricular fibrillation can also be detected.

In another exemplary aspect, once the PDT (Pulse Duration Time) amplitude is derived from the PPG signal, heart rate variability (HRV) in a patient's body can be detected. It has been suggested that ratio of pulse interval to its systolic amplitude could provide an understanding of the properties of the patient's cardiovascular system. When the HRV derived using the pulse interval in PPG signals have been compared with the HRV using R-R intervals in ECG signals, the results have demonstrated that HRV in PPG and ECG signals are highly correlated. They strongly suggested that PPG signals could be used as an alternative measurement of HRV.

In another exemplary aspect, once the SDPT (Systolic to diastolic peak difference) is derived from the PPG signal, the Large Artery Stiffness Index in a patient's body can be detected. Time delay between the systolic and diastolic peaks, or, in the absence of a second peak, the point of inflection is related to the transit time of pressure waves from the root of the subclavian artery to the apparent site of reflection and back to the subclavian artery. This path length can be assumed to be proportional to subject height.

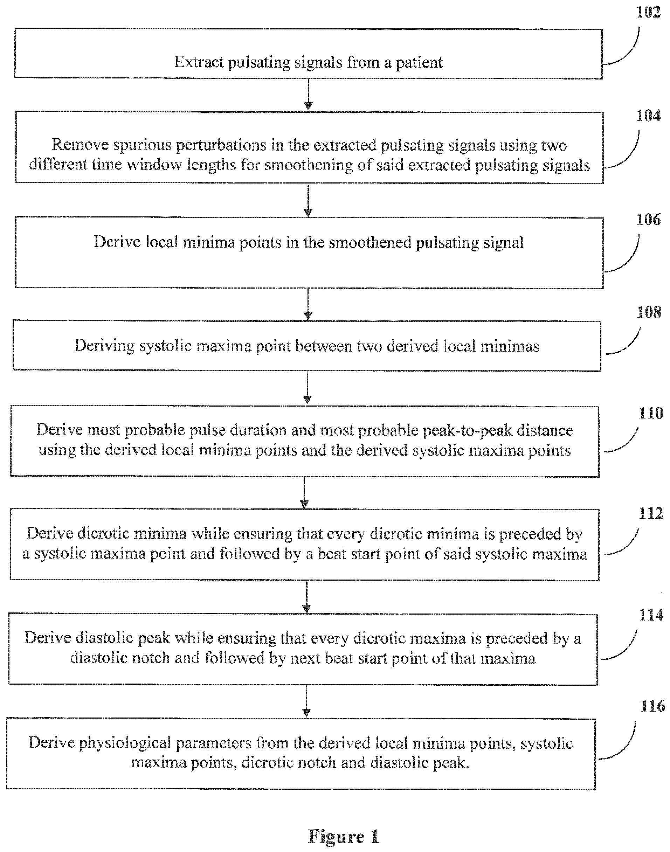

Referring to Figure 1, it is a flow chart illustrating a method for deriving physiological parameters from pulsating signals with reduced error.

The process starts at step 102, pulsating signals are extracted from a patient. At the step 104, spurious perturbations in the extracted pulsating signals are removed. At step 106, local minima points are derived in the smoothened pulsating signal. At step 108, systolic maxima point between two derived local minimas are derived. At step 110, most probable pulse duration and most probable peak-to-peak distance are derived using the derived local minima points and the derived systolic maxima points. At step 112, dicrotic minima is derived while ensuring that every dicrotic minima is preceded by a systolic maxima point and followed by a beat start point of said systolic maxima. At step 114, diastolic peak is derived while ensuring that every dicrotic maxima is preceded by a diastolic notch and followed by next beat tart point of that maxima and at step 116, physiological parameters are derived from the derived local minima points, systolic maxima points, dicrotic notch and diastolic peak.

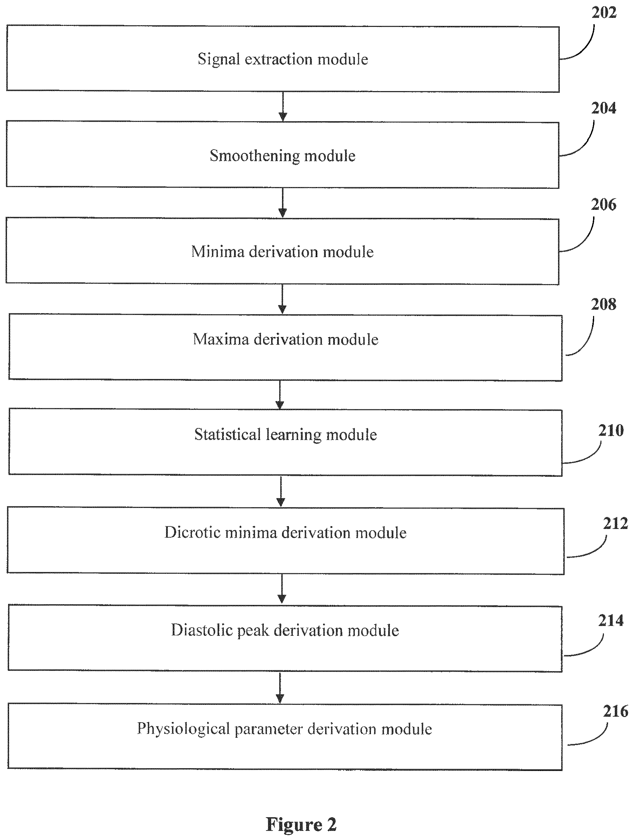

Referring to Figure 2, it is a block diagram illustrating system architecture for deriving physiological parameters from pulsating signals with reduced error.

In an embodiment of the present invention, a system (200) for deriving a plurality of physiological parameters from pulsating signals with reduced error for monitoring cardiac health of a patient comprises of a signal extraction module (202), a smoothening module (204), a minima derivation module (206), a maxima derivation module (208), a statistical learning module (210), a dicrotic minima derivation module (212), a diastolic peak derivation module (214) and a physiological parameter derivation module (216).

The method described herein is applicable for any pulsating signals. The method derives various time series and signal properties and co-relates them with various physiological parameters. It indicates physiological abnormality in a noninvasive way and performs analytics with reduced errors and optimized resource usage. Thus, in an exemplary embodiment, the applicable pulsating signal is selected from photoplethysmogram (PPG) signals and arterial blood pressure (ABP) signals.

In another embodiment of the present invention, the signal extraction module (202) consists of a mobile communication device or a wearable device. In an exemplary embodiment of the present invention, wherein photoplethysmogram (PPG) signal is used as the pulsating signal, an image capturing device is coupled with the mobile communication device for extracting photoplethysmogram (PPG) signals from the patient. The photoplethysmogram (PPG) signals are extracted from patient's peripheral body parts selected from a group comprising but not limited to finger, ear, forehead and toe. The mobile communication device captures photoplethysmogram signal in reflective mode and the mobile communication device is selected from a group comprising of smart phone, mobile phone, laptop, tablet, and personal digital assistant. The image capturing device coupled with the mobile communication device is a camera and has a light emitting source for extracting photoplethysmogram signals from the patient's peripheral body parts selected from a group comprising but not limited to finger, ear, toe; forehead, thereby, obtaining a video sequence of the light, reflected from patient's peripheral body parts.

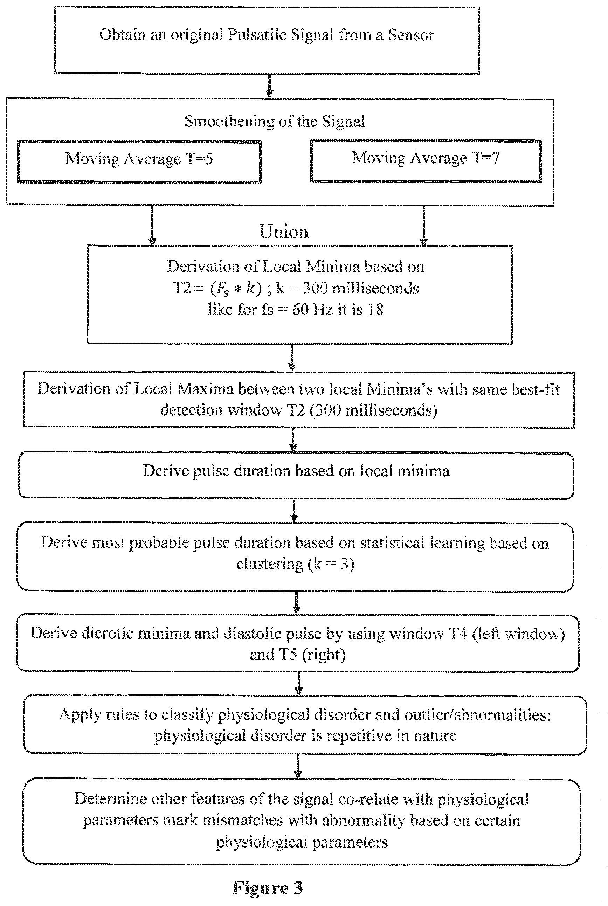

In another embodiment of the present invention, in the smoothening module (204), spurious perturbations in the extracted pulsating signals are removed by using two different time window lengths for smoothening of said extracted pulsating signals. Smoothening is performed to remove inequalities in the said extracted pulsating signals by taking union of two MA (moving average) windows (MA= 5 and MA= 7) instead of a single window, in accordance with figure 3. Signals with union operation after MA (moving average):

$$S1 = X1, X2, \ldots\ldots\ldots\ldots\ldots XM ;$$

set of Local Minima obtained taking T1=5

$$S2 = Y1, Y2, \ldots\ldots\ldots\ldots\ldots\ldots YN;$$

set of Local Minima obtained taking T1=7

$$S = S1 \cup S2$$

Arranging S in increasing order.

$$\text{If } (|a_i - a_{i+1}| <= 2)$$

$$\{$$

$$\text{Replace both points } (a_i, a_{i+1}) \text{ by } ((a_i + a_{i+1})/2)$$

$$\}$$

In another embodiment of the present invention, in the minima derivation module (206), local minima points are derived in the smoothened pulsating signals based on the discrete nature of said pulsating signals and strictly rising and falling edge of said pulsating signals and union of minima points derived from the smoothened pulsating signals with two different window sizes, in accordance with figure 3. Derivation of Local Minima is based $T2 = (Fs * k)$; k= 300 milliseconds. Mathematical equations to derive local minima:

$$Y_{t0-10} > Y_{t0-t9} > \ldots\ldots\ldots\ldots\ldots > Y_{t0} < Y_{t0+1} < \ldots\ldots\ldots\ldots < Y_{t0+10}$$

Consecutive local minima's $|Y_{tn-1} - Y_{tn}| > T3$; $T3 = (F_s * k)$ ; k = 300 milliseconds where T3 denotes the threshold. The local minima is derived by using the following method:

Input:

T2: Window size
T3: Distance between two minima
$\tilde{D}_{MA}$ : Smoothed signal $x_k$ with $k$ = 1,2,3, ...$N$: N: $length(D)$
Output: MI: Minima location.
$M$:= Detecting Indices of $\tilde{D}$ such that:

$$\tilde{x}_{k-\frac{T2}{2}} > \tilde{x}_{k-\frac{T2}{2}+1} \ldots > \tilde{x}_{k-1} > \tilde{x}_k < \tilde{x}_{k+1} \ldots < \tilde{x}_{k+\frac{T2}{2}}$$

MI := Discard indices $M_k$ in $M$ such that $|M_k - M_{k-1}| < T3$

In another embodiment of the present invention, in the maxima derivation module (208), systolic maxima point between two derived local minimas are derived based on the discrete nature of said pulsating signals, strictly rising and falling edge of said pulsating signals and union of maxima points derived from the smoothened pulsating signals with two different window sizes instead of one window as used in the traditional methods, in accordance with figure 3. The derivation of Local Maximas based on deriving a local maxina between two local minimas with same best-fit detection window T2 (300 milliseconds). The systolic maxima point between two derived local minima points is derived and similar steps are performed as obtaining local minima points to result in the final set of systolic maxima points.

In another embodiment of the present invention, in the statistical learning module (210), most probable pulse duration and most probable peak-to-peak distance are derived using the derived local minima points and the derived systolic maxima points, in accordance with figure 3. The said derivation of most probable pulse duration and most probable peak-to-peak distance is based on the univariate nature of said pulsating signals. In this module, first pulse duration is derived based on local minima. Next, most probable pulse duration is derived based on statistical learning based on clustering (k = 3) while exploiting the uni-dimensional nature of the signal.

In another embodiment of the present invention, in the dicrotic minima derivation module (212), dicrotic minima is derived based on the discrete nature of said pulsating signals, strictly rising and falling edge of said pulsating signals and union of the local minima points derived from two different smoothened pulsating signals with two different sizes ensuring that every dicrotic minima is preceded by a systolic maxima point and followed by a beat start point of said systolic maxima. The following method is followed for dicrotic minima derivation:

Function p = Detect dicrotic minima

Input: $\varphi_{min}$, $\varphi_{max}$:

$win_l$: Window size on the left
$win_T$: Window size on the right
$\tilde{D}_{MA}$ : Smoothed signal $x_k$ with $k = 1,2,3..... N$; N: *length* ($D\varphi_{min}$: Final set of minima obtained

Output:

DI: Dicrotic indices
$M_{dc}$:= Find Indices of $\tilde{D}$ such that:

$$\tilde{x}_{k-win_l} > \tilde{x}_{k-win_l+1} \cdots > \tilde{x}_{k-1} > \tilde{x}_k < \tilde{x}_{k+1} \cdots < \tilde{x}_{k+win_T}$$

$M^l_{dc}$ = Filter $M_{dc}$: to ensure that every dicrotic minima is preceded by a maxima in $\varphi_{max}$ and followed by the next minima in $\varphi_{min}$ of that maxima.

$$DI = M'_{dc} - \varphi_{min}$$

In another embodiment of the present invention, in the diastolic peak derivation module (214), diastolic peak is derived based on the discrete nature of said pulsating signals, strictly rising and falling edge of said pulsating signals and union of the systolic maxima points derived from two different smoothened pulsating signals with two different sizes ensuring that every dicrotic maxima is preceded by a diastolic notch and followed by next beat start point of that maxima. Every diastolic peak should be situated between a dicrotic minima and the immediately following minima point and derived by using the similar steps as in dicrotic minima points.

In another embodiment of the present invention, in the physiological parameter derivation module (216), physiological parameters are derived from the derived local minima points, systolic maxima points, dicrotic notch and diastolic peak. The physiological parameter derivation module (216) further consists of marking physiological abnormalities based on the derived physiological parameters and the marking of physiological abnormalities is performed using a predefined set of rules. The predefined set of rules is used to classify between abnormalities arising out of physiological parameter disorder and anomaly outliers, wherein the physiological parameter disorders are repetitive in nature.

**Claims**

1. A method for deriving a plurality of physiological parameters from pulsating signals for monitoring cardiac health of a patient; said method comprising

   a. extracting pulsating signals from the patient using a signal extraction module (202);
   b. removing spurious perturbations in the extracted pulsating signals using two different time window lengths for smoothening of said extracted pulsating signals by using a smoothening module (204);
   c. deriving local minima points in the smoothened pulsating signals based on the discrete nature of said pulsating signals and strictly rising and falling edge of said pulsating signals and union of minima points derived from the smoothened pulsating signals with two different sizes using a minima derivation module (206);
   d. deriving systolic maxima point between two derived local minimas based on the discrete nature of said pulsating signals, strictly rising and falling edge of said pulsating signals and union of maxima points derived from the smoothened pulsating signals with two different sizes using a maxima derivation module (208);
   e. deriving most probable pulse duration and most probable peak-to-peak distance using the derived local minima points and the derived systolic maxima points and based on the univariate nature of said pulsating signals by using a statistical learning module (210);
   f. deriving dicrotic minima based on the discrete nature of said pulsating signals, strictly rising and falling edge of said pulsating signals and union of the local minima points derived from two different smoothened pulsating signals with two different sizes ensuring that every dicrotic minima is preceded by a systolic maxima point and followed by a beat start point of said systolic maxima by using a dicrotic minima derivation module (212);
   g. deriving diastolic peak based on the discrete nature of said pulsating signals, strictly rising and falling edge of said pulsating signals and union of the systolic maxima points derived from two different smoothened pulsating signals with two different sizes ensuring that every dicrotic maxima is preceded by a diastolic notch and followed by next beat start point of that maxima by using a diastolic peak derivation module (214); and
   h. deriving physiological parameters from the derived local minima points, systolic maxima points, dicrotic notch and diastolic peak using a physiological parameter derivation module (216);

2. The method as claimed in claim 1, wherein said pulsating signals is selected from photoplethysmogram (PPG) signals and arterial blood pressure (ABP) signals.

3. The method as claimed in claim 1, wherein the signal extraction module (202) consists of a mobile communication device or a wearable device.

4. The method as claimed in claim 1, wherein the smoothening of the extracted pulsating signals is performed by taking union of two moving average windows.

5. The method as claimed in claim 1, wherein the derivation of local minima points in the smoothened pulsating signals is a time window based process.

6. The method as claimed in claim 1, wherein the derivation of best fit window is used as 300 milliseconds.

7. The method as claimed in claim 1, wherein the derivation of systolic maxima between two derived local minimas is a time window based process.

8. The method as claimed in claim 1 further consists of marking physiological abnormalities based on the derived physiological parameters.

9. The method as claimed in claim 8, wherein said marking of physiological abnormalities is performed using a predefined set of rules.

10. The method as claimed in claim 9, the predefined set of rules is used to classify between abnormalities arising out of physiological parameter disorder and anomaly outliers.

11. The method as claimed in claim 10, the physiological parameter disorders are repetitive in nature.

12. A system for deriving a plurality of physiological parameters from pulsating signals for monitoring cardiac health of a patient; said system comprising:

a. a signal extraction module (202) adapted for extracting pulsating signals from the patient;

b. a smoothening module (204) adapted for removing spurious perturbations in the extracted pulsating signals using two different time window lengths for smoothening of said extracted pulsating signals;

c. a minima derivation module (206) adapted for deriving local minima points in the smoothened pulsating signals based on the discrete nature of said pulsating signals and strictly rising and falling edge of said pulsating signals and union of minima points derived from the smoothened pulsating signals with two different sizes;

d. a maxima derivation module (208) adapted for deriving systolic maxima point between two derived local minimas based on the discrete nature of said pulsating signals, strictly rising and falling edge of said pulsating signals and union of maxima points derived from the smoothened pulsating signals with two different sizes;

e. a statistical learning module (210) adapted for deriving most probable pulse duration and most probable peak-to-peak distance using the derived local minima points and the derived systolic maxima points and based on the univariate nature of said pulsating signals;

f. a dicrotic minima derivation module (212) adapted for deriving dicrotic minima based on the discrete nature of said pulsating signals, strictly rising and falling edge of said pulsating signals and union of the local minima points derived from two different smoothened pulsating signals with two different sizes ensuring that every dicrotic minima is preceded by a systolic maxima point and followed by a beat start point of said systolic maxima;

g. a diastolic peak derivation module (214) adapted for deriving diastolic peak based on the discrete nature of said pulsating signals, strictly rising and falling edge of said pulsating signals and union of the systolic maxima points derived from two different smoothened pulsating signals with two different sizes ensuring that every dicrotic maxima is preceded by a diastolic notch and followed by next beat start point of that maxima; and

h. a physiological parameter derivation module (216) adapted for deriving physiological parameters from the derived local minima points, systolic maxima points, dicrotic notch and diastolic peak;

102

Extract pulsating signals from a patient

104

Remove spurious perturbations in the extracted pulsating signals using two different time window lengths for smoothening of said extracted pulsating signals

106

Derive local minima points in the smoothened pulsating signal

108

Deriving systolic maxima point between two derived local minimas

110

Derive most probable pulse duration and most probable peak-to-peak distance using the derived local minima points and the derived systolic maxima points

112

Derive dicrotic minima while ensuring that every dicrotic minima is preceded by a systolic maxima point and followed by a beat start point of said systolic maxima

114

Derive diastolic peak while ensuring that every dicrotic maxima is preceded by a diastolic notch and followed by next beat start point of that maxima

116

Derive physiological parameters from the derived local minima points, systolic maxima points, dicrotic notch and diastolic peak.

**Figure 1**

**Figure 2**

Obtain an original Pulsatile Signal from a Sensor

Smoothening of the Signal

Moving Average T=5

Moving Average T=7

Union

Derivation of Local Minima based on
T2= $(F_s * k)$ ; k = 300 milliseconds
like for fs = 60 Hz it is 18

Derivation of Local Maxima between two local Minima's with same best-fit
detection window T2 (300 milliseconds)

Derive pulse duration based on local minima

Derive most probable pulse duration based on statistical learning based on
clustering (k = 3)

Derive dicrotic minima and diastolic pulse by using window T4 (left window)
and T5 (right)

Apply rules to classify physiological disorder and outlier/abnormalities:
physiological disorder is repetitive in nature

Determine other features of the signal co-relate with physiological
parameters mark mismatches with abnormality based on certain
physiological parameters

**Figure 3**

Europäisches
Patentamt

European
Patent Office

Office européen
des brevets

# EUROPEAN SEARCH REPORT

Application Number

EP 17 16 3197

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SRINIVAS KUNTAMALLA ET AL: "An Efficient and Automatic Systolic Peak Detection Algorithm for Photoplethysmographic Signals", INTERNATIONAL JOURNAL OF COMPUTER APPLICATIONS, vol. 97, no. 19, 1 July 2014 (2014-07-01), pages 18-23, XP055412228, DOI: 10.5120/17115-7686 * abstract * * paragraph [0001] - paragraph [02.4] * * figures 1,2 * | 1-12 | INV. A61B5/00 A61B5/024 |
| A | ABOY M ET AL: "Automatic detection algorithm for physiologic pressure signal components", SECOND JOINT EMBS-BMES CONFERENCE 2002. CONFERENCE PROCEEDINGS. 24TH. ANNUAL INTERNATIONAL CONFERENCE OF THE ENGINEERING IN MEDICINE AND BIOLOGY SOCIETY. ANNUAL FALL MEETING OF THE BIOMEDICAL ENGINEERING SOCIETY. HOUSTON, TX, OCT. 23 - 26, 2002; [ANN, vol. 1, 23 October 2002 (2002-10-23), pages 196-197, XP010621273, DOI: 10.1109/IEMBS.2002.1134453 ISBN: 978-0-7803-7612-0 * abstract * * paragraph [000I] - paragraph [00II] * * figures 1,2 * | 1-12 | |

-/--

TECHNICAL FIELDS SEARCHED (IPC)

A61B

The present search report has been drawn up for all claims

| Place of search | Date of completion of the search | Examiner |
|---|---|---|
| The Hague | 6 October 2017 | Van der Haegen, D |

EPO FORM 1503 03.82 (P04C01)

Europäisches
Patentamt

European
Patent Office

Office européen
des brevets

**EUROPEAN SEARCH REPORT**

Application Number

EP 17 16 3197

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/058231 A1 (WATSON JAMES [GB]) 27 February 2014 (2014-02-27) * abstract * * paragraph [0002] - paragraph [0009] * * paragraph [0035] - paragraph [0038] * * paragraph [0053] - paragraph [0058] * * paragraph [0063] - paragraph [0082] * * figures 5-7 * | 1-12 | |
| A | WO 2015/142603 A1 (THE FEINSTEIN INST MEDICAL RES [US]; JERUSALEM COLLEGE TECH [IL]) 24 September 2015 (2015-09-24) * paragraph [0010] * * paragraph [0038] * | 1-12 | |
| A | WO 02/071936 A1 (AUCKLAND UNISERVICES LTD [NZ]; MALPAS SIMON CHARLES [NZ]; NAVAKATIKYAN) 19 September 2002 (2002-09-19) * page 1, line 1 - line 8 * * page 2, line 27 - page 7, line 18 * * page 11, line 18 - page 16, line 32 * * page 22, line 29 - page 23, line 27 * * figures 1,5,11-13,18,27,28 * | 1-12 | TECHNICAL FIELDS SEARCHED (IPC) |

The present search report has been drawn up for all claims

| Place of search | Date of completion of the search | Examiner |
|---|---|---|
| The Hague | 6 October 2017 | Van der Haegen, D |

EPO FORM 1503 03.82 (P04C01)

## ANNEX TO THE EUROPEAN SEARCH REPORT
## ON EUROPEAN PATENT APPLICATION NO.

EP 17 16 3197

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2017

| Patent document cited in search report | | | Publication date | Patent family member(s) | | | Publication date |
|---|---|---|---|---|---|---|---|
| US 2014058231 | A1 | | 27-02-2014 | US | 2009326395 A1 | | 31-12-2009 |
| | | | | US | 2014058231 A1 | | 27-02-2014 |
| WO 2015142603 | A1 | | 24-09-2015 | US | 2017164842 A1 | | 15-06-2017 |
| | | | | WO | 2015142603 A1 | | 24-09-2015 |
| WO 02071936 | A1 | | 19-09-2002 | AU | 2002248091 B2 | | 10-08-2006 |
| | | | | EP | 1372473 A1 | | 02-01-2004 |
| | | | | US | 2004097814 A1 | | 20-05-2004 |
| | | | | WO | 02071936 A1 | | 19-09-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 20162108713

- IN 31052016